# EUROPEAN PATENT SPECIFICATION

(11) **EP 4 335 479 B1**
(45) Date of publication and mention of the grant of the patent: **13.05.2026**
(21) Application number: 23194140.2
(22) Date of filing: 30.08.2023
(51) Int. Cl.: A61M 25/00, B29C 65/00

(54) **CATHETER**
KATHETER
CATHÉTER

(30) Priority: 07.09.2022 JP 2022141908
(43) Date of publication of application: 13.03.2024
(73) Proprietor: ASAHI INTECC CO., LTD., Seto-shi, Aichi 489-0071 (JP)
(72) Inventor: KANEKO, Yoshiki, Seto-shi, Aichi 489-0071 (JP)
(74) Representative: Winter, Brandl - Partnerschaft mbB

(56) References cited:
- EP-A1- 2 944 344
- WO-A1-2006/126642
- WO-A1-2018/008272
- JP-A- 2009 082 566

## Description

### TECHNICAL FIELD

The technology disclosed herein relates to a catheter.

### BACKGROUND ART

A catheter is an elongated medical device to be inserted for use into a biological lumen such as a blood vessel to diagnose or treat the biological lumen.

A catheter having two or more lumens is known for the purposes such as for improving the vascular selectivity of a guide wire. For example, a catheter of this type has a configuration in which a plurality of inner tubes each having a lumen are inserted into an outer tube and welded to the outer tube (for example, see JP 2015 - 213 684 A). When the tubes of the catheter are welded together, the wall thickness of the tubes may become thinner and the strength of the tubes may be decreased. Reduced strength of the tubes each having a lumen may, for example, cause a guide wire to unintentionally protrude out of a lumen. JP 2009 - 082 566 A discloses a catheter comprising a first lumen and a second lumen, wherein at least the second lumen is composed of an inner layer, a reinforcing layer, and an outer layer. EP 2 944 344 A1 discloses a catheter including an outer tube, a first inner tube, a second inner tube, and a reinforcing member. Protective films cover the outer periphery of the inner tubes. Welded parts connect the outer tube, the protective films, and the inner tubes to one another. WO 2006 / 126 642 A1 discloses a catheter having a distal shaft, a proximal shaft, a metallic core wire, and a hub. The distal shaft has a dual lumen structure having a first lumen and a second lumen. The distal shaft further comprises inner layers and an outer layer of the shaft forming the inner surfaces of the two lumens of the first lumen and the second lumen. WO 2018 / 008272 A1 discloses a catheter comprising a distal shaft having a first tube including a first lumen, a second tube including a second lumen and a reinforcing layer.

Conventionally, a catheter having a protective film placed between an inner tube and an outer tube or between a plurality of inner tubes has been proposed to prevent decreased wall thickness due to inter-tube welding.

### SUMMARY OF INVENTION

### Technical Problem

The invention is according to claim 1. Preferred embodiments of the invention are set forth in the dependent claims.

In the aforementioned configurations of the conventional catheters, there is room for improving the strength of a tube having a lumen.

Disclosed herein is a technology which can solve the above problem. This problem is solved by the subject matter of the independent claim. Further aspects are disclosed in the dependent claims.

### Solution to Problem

The technology disclosed herein can be implemented, for example, according to the following aspects.
(1) A catheter disclosed herein comprises a first tube having a first lumen, and a second tube having a second lumen. The first tube has an inner layer corresponding to the innermost layer of the first lumen, an outer layer surrounding the inner layer, and a reinforcing layer sandwiched between the inner and the outer layer.
   According to the present catheter, the strength of the first tube having the first lumen can be improved by means of the reinforcing layer sandwiched between the inner layer and the outer layer. This can, for example, prevent a guide wire from unintentionally protruding out of the first lumen, or can prevent a guide wire from being stuck due to a collapse of the first lumen.
(2) The above catheter further comprises a connecting layer covering at least a portion of outer peripheral surfaces of the first tube and the second tube to connect the first tube and the second tube, and having a melting point lower than those of the inner layer and the outer layer. This configuration can prevent the inner layer and the outer layer for the first tube from becoming thinner due to welding, effectively increasing the strength of the first tube, and can also prevent the reinforcing layer sandwiched between the inner layer and the outer layer from being exposed. As further, a core wire is embedded in the connection layer. As further, in a cross section of the catheter, the first tube and the second tube are exposed to outside of the connection layer at both ends in a direction where the first tube and the second tube are aligned side by side.
(3) In the above catheter, an outer periphery of the catheter may be elliptical in a transverse section. This configuration can improve the passability through a lesion site and the operability of a concomitantly-used device as compared to a configuration in which an outer periphery is circular in a transverse section.
(4) In the above catheter, the reinforcing layer may be a coil. This configuration enables the coil as a reinforcing layer to improve the strength of the first tube while ensuring the flexibility required for the catheter.
(5) In the above catheter, the reinforcing layer may be a first reinforcing layer, and the inner layer may be a first inner layer, and the outer layer may be a first outer layer, and the second tube may have a second inner layer corresponding to the innermost layer of the second lumen, a second outer layer surrounding the second inner layer, and a second reinforcing layer sandwiched between the second inner layer and the second outer layer. This configuration can improve the strength of the second tube having the second lumen by means of the second reinforcing layer sandwiched between the second inner layer and the second outer layer. This can, for example, prevent a guide wire from unintentionally protruding out of the second lumen, or can prevent a guide wire from being stuck due to a collapse of the second lumen.

The technology disclosed herein can be implemented according to various aspects, for example, according to the aspects of catheters, medical devices including catheters, methods of manufacturing them or methods of using them, and the like.

### BRIEF DESCRIPTION OF THE DRAWINGS

FIG 1 shows a diagram schematically illustrating a side view of a catheter according to the present embodiment.
FIG 2 shows a diagram schematically illustrating a longitudinal sectional view of the catheter according to the present embodiment.
FIG 3 shows a diagram schematically illustrating a transverse sectional view of the catheter according to the present embodiment.

### DESCRIPTION OF EMBODIMENTS

### A. Embodiments:

### A-1. Configuration of catheter 10:

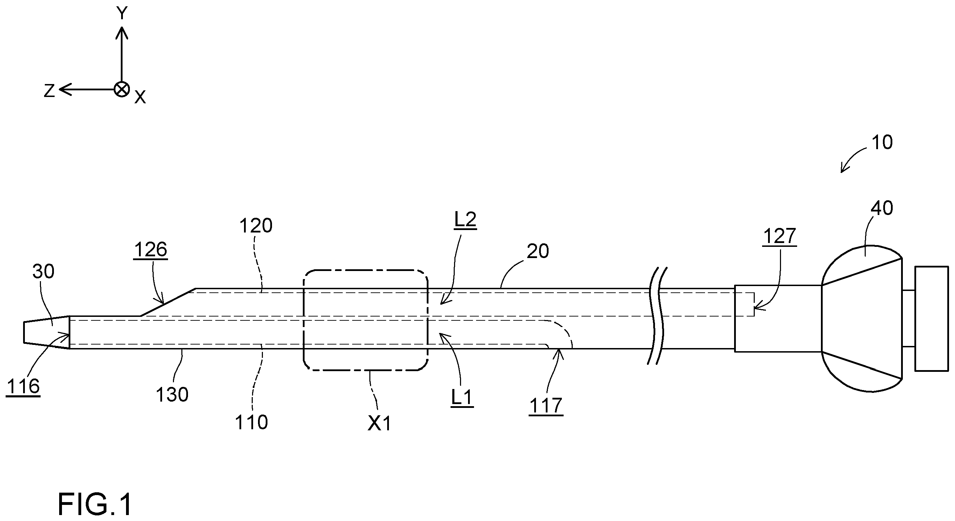

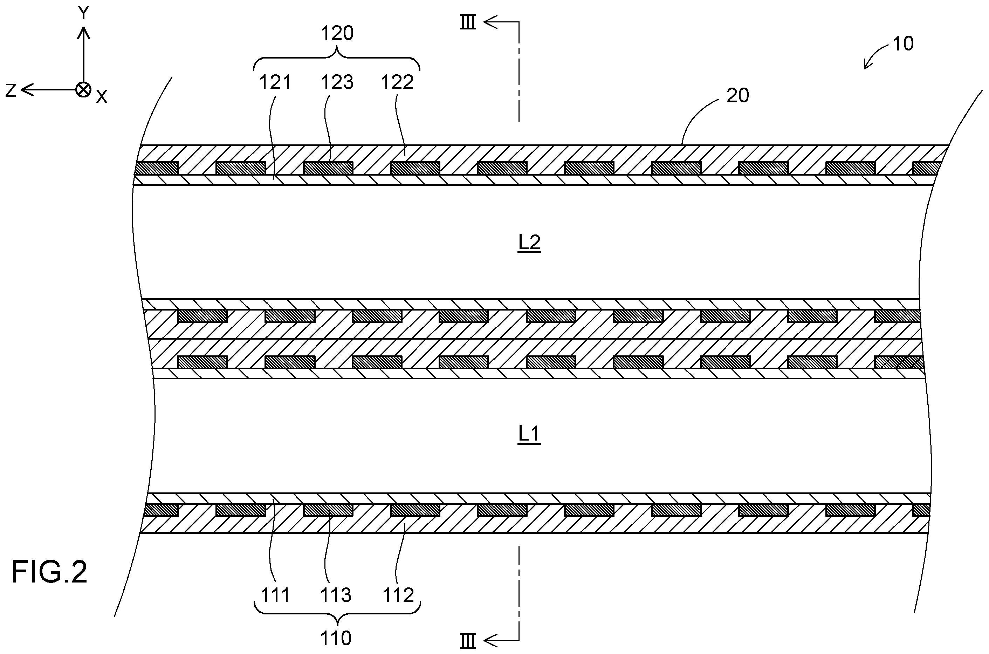

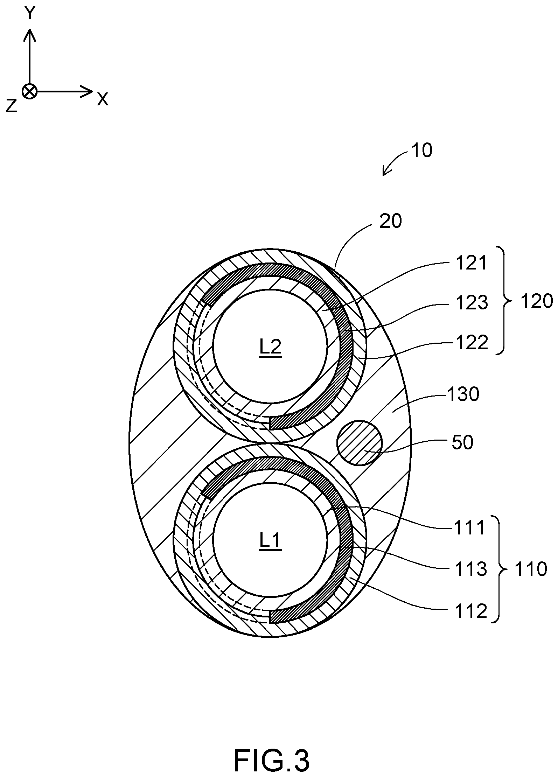

FIGs. 1 to 3 show diagrams schematically illustrating the configuration of a catheter 10 according to the present embodiment. FIG. 1 shows a side view of the catheter 10, and FIG 2 shows a longitudinal sectional view (the YZ cross section) of a portion of the catheter 10 (the X1 portion in FIG 1), and FIG. 3 shows a transverse sectional view (the XY cross section) of the catheter 10 at the III-III position in FIG 2. In each of the figures, the positive direction of the Z axis corresponds to a distal end side (distal side) to be inserted into the body, and the negative direction of the Z-axis corresponds to a proximal end side (proximal side) to be operated by an operator such as a medical practitioner. Although each of the figures represents a state where the catheter 10 is extended in linear manner substantially parallel to the Z-axis direction as a whole, the configuration of at least a portion of the catheter 10 is flexible enough to be bent. It is noted that as used herein with reference to the catheter 10 and each component member thereof, a tip in the distal end side is referred to as a "distal end", and the distal end and the vicinity thereof are referred to as a "distal end portion". A tip of the proximal end side is referred to as a "proximal end", and the proximal end and the vicinity thereof are referred to as a "proximal end portion".

The catheter 10 is an elongated medical device to be inserted for use into a biological lumen such as a blood vessel to diagnose or treat the biological lumen. As shown in FIG 1, the catheter 10 has a shaft 20, a distal tip 30, and a connector 40.

The shaft 20 is an elongated tubular member. As shown in FIG. 3, the outer periphery is of an elliptical shape in a transverse section of a prescribed portion (the X1 portion in FIG 1) of the shaft 20. The elliptical shape as used herein is not limited to mathematically precise elliptical shapes, but includes any shapes in which a circle is distorted in at least one direction. The shape (length and diameter) of the shaft 20 is suitably designed depending on the purposes and positions of use and the like of the catheter 10.

Two lumens (a first lumen L1 and a second lumen L2) extending along the longitudinal direction of the shaft 20 are formed in the shaft 20. The first lumen L1 is a lumen for a guide wire. The second lumen L2 is a lumen for a guide wire. An opening 116 at the distal end side of the first lumen L1 is arranged at the distal end of the shaft 20 and connected to the distal tip 30, while an opening 117 at the proximal end side of the first lumen L1 is arranged on the peripheral wall at the middle portion of the shaft 20 and is open to the outside of the catheter 10. That is, the first lumen L1 is a lumen of the rapid exchange (RX) type formed in a region from the distal end portion to the middle portion of the shaft 20. Meanwhile, an opening 126 at the distal end side of the second lumen L2 is arranged at the distal end portion of the shaft 20, and an opening 127 at the proximal end side of the second lumen L2 is arranged at the proximal end of the shaft 20, and connected to the connector 40. That is, the second lumen L2 is a lumen of the over-the-wire (OTW) type formed throughout substantially the entire length (a region from the distal end portion to the proximal end portion) of the shaft 20. The configuration of the shaft 20 will be described in detail below.

The distal tip 30 is a tubular (hollow) member, and joined to the distal end of the shaft 20. A method of joining the distal tip 30 to the shaft 20 maybe, for example, welding, adhesion by adhesives, or the like. An inner cavity extending in the longitudinal direction of the catheter 10 is formed in the distal tip 30, and the inner cavity is in communication with the first lumen L1 formed in the shaft 20. The distal tip 30, for example, is tapered with the outer diameter decreasing progressively toward the distal end side.

As a material for the distal tip 30, resin materials may be used, for example, polyurethane, polyurethane elastomer, polyamide, and polyamide elastomer, and the like. Powders of radiopaque materials (e.g., tungsten, gold, platinum, and the like) may be mixed with these resin materials to make the resins visible under exposure of X-rays. Alternatively, a marker formed of a radiopaque material may be provided in the distal tip 30.

The connector 40 is a member to be held by an operator such as a medical practitioner, and connected to the proximal end of the shaft 20. An inner cavity extending in the longitudinal direction of the catheter 10 is formed in the connector 40, and the inner cavity is in communication with the second lumen L2 formed in the shaft 20.

Next, the configuration of the shaft 20 will be described in more detail. The shaft 20 has a first tube 110, a second tube 120, and a connection layer 130.

The first tube 110 is a tubular member having the first lumen L1. The first tube 110 is arranged in a region from the distal end portion to the middle portion of the shaft 20. Consequently, the first lumen L1 is formed in the region from the distal end portion to the middle portion of the shaft 20, as described above.

As shown in FIG 2 and FIG. 3, the first tube 110 has a first inner layer 111 corresponding to the innermost layer of the first lumen L1, a first outer layer 112 surrounding the first inner layer 111, and a first reinforcing layer 113 sandwiched between the first inner layer 111 and the first outer layer 112. The first inner layer 111 and the first outer layer 112 are, for example, tubular members each having a circular transverse section. The first reinforcing layer 113 is a coil spirally wound around the outer peripheral surface of the first inner layer 111. In the present embodiment, the first reinforcing layer 113 is a sparsely wound coil. The first reinforcing layer 113 is arranged throughout the entire length of the first tube 110. However, the first reinforcing layer 113 may only be arranged at a portion in the longitudinal direction (for example, a portion of the distal end side) of the first tube 110.

The second tube 120 is a tubular member having the second lumen L2. The second tube 120 is arranged throughout substantially the entire length (a region from the distal end portion to the proximal end portion) of the shaft 20. Consequently, the second lumen L2 is formed throughout substantially the entire length of the shaft 20, as described above.

As shown in FIG. 2 and FIG. 3, the second tube 120 has a second inner layer 121 corresponding to the innermost layer of the second lumen L2, a second outer layer 122 surrounding the second inner layer 121, and a second reinforcing layer 123 sandwiched between the second inner layer 121 and the second outer layer 122. The second inner layer 121 and the second outer layer 122 are, for example, tubular members each having a circular transverse section. The second reinforcing layer 123 is a coil spirally wound around the outer peripheral surface of the second inner layer 121. In the present embodiment, the second reinforcing layer 123 is a sparsely wound coil. The second reinforcing layer 123 is arranged throughout the entire length of the second tube 120. However, the second reinforcing layer 123 may only be arranged at a portion in the longitudinal direction (for example, a portion of the distal end side) of the second tube 120.

As shown in FIG 3, the first tube 110 and the second tube 120 are arranged side by side along the Y axis direction in a transverse section (the XY cross section) of the shaft 20. The first tube 110 and the second tube 120 are continuously in contract with each other along the longitudinal direction of the shaft 20. In the present embodiment, the first tube 110 and the second tube 120 have substantially the same outer diameter and inner diameter.

As materials for the first inner layer 111 and the first outer layer 112 of the first tube 110, and the second inner layer 121 and the second outer layer 122 of the second tube 120, resin materials may be used, for example, polyimide, polyamide, polyamide elastomer, polyolefin, polyester, polyester elastomer, nylon, polytetrafluoroethylene, and the like. The materials of the above layers may be the same or different from each other. As materials for the first reinforcing layer 113 and the second reinforcing layer 123, for example, metal materials such as stainless steel and Ni-Ti alloys, and resin materials such as PEEK may be used.

The connection layer 130 is a layer covering at least a portion of the outer peripheral surfaces of the first tube 110 and the second tube 120 to connect the first tube 110 and the second tube 120. As shown in FIG 3, in the transverse section (the XY cross section) of the shaft 20 according to the present embodiment, the first tube 110 and the second tube 120 are exposed to the outside without being covered with the connection layer 130 at the both ends in a direction (the Y-axis direction) where the first tube 110 and the second tube 120 are aligned side by side. It is noted that the entire outer peripheral surfaces of the first tube 110 and the second tube 120 may be covered with the connection layer 130. A core wire 50 is further embedded in the connection layer 130. The core wire 50 extends along with the longitudinal direction of the shaft 20.

As a material for the connection layer 130, resin materials may be used, for example, polyamide, polyamide elastomer, polyolefin, polyester, polyester elastomer, nylon, and the like. It is noted that the melting point of the connection layer 130 is preferably lower than those of the first inner layer 111 and the first outer layer 112 of the first tube 110 and the second inner layer 121 and the second outer layer 122 of the second tube 120. As a material for the core wire 50, metal materials may be used, for example, stainless steel, Ni-Ti alloys, cobalt-chromium alloys, and the like.

The shaft 20 having such a configuration can be, for example, manufactured as follows. First, a cored bar is coated with the first inner layer 111 for the first tube 110, and a coil as the first reinforcing layer 113 is wound around the outer peripheral surface of the first inner layer 111. Then, the first inner layer 111 and the first reinforcing layer 113 are coated and covered with the first outer layer 112, and the first outer layer 112 welded under compression with a contraction tube. Thereby, the first tube 110 is formed having a configuration in which the first reinforcing layer 113 is sandwiched between the first inner layer 111 and the first outer layer 112. The second tube 120 is prepared by an analogous method.

Next, the first tube 110, the second tube 120, and the core wire 50 are coated and integrally covered with a material for forming the connection layer 130, and the material welded under compression with a contraction tube. Thereby, the connection layer 130 is formed covering at least a portion of the outer peripheral surfaces of the first tube 110 and the second tube 120 to connect the first tube 110 and the second tube 120. The welding temperature at this time is set to, for example, a temperature higher than the melting point of the connection layer 130, but lower than the melting points of the first inner layer 111 and the first outer layer 112 for first tube 110 and the second inner layer 121 and the second outer layer 122 for second tube 120. When the welding temperature is set in this manner, the first inner layer 111 and the first outer layer 112 for the first tube 110 and the second inner layer 121 and the second outer layer 122 for the second tube 120 can maintain their shapes (outer diameter and wall thickness) during welding without melting. On the other hand, the connection layer 130 melts, and flows out from the both ends in a direction where the first tube 110 and the second tube 120 are aligned side by side (the Y-axis direction) as shown in FIG 3. As a result, the resulting shaft 20 has an outer periphery elliptical in a transverse section.

### A-2. Advantageous effects of the present embodiment:

As described above, the catheter 10 according to the present embodiment comprises the first tube 110 having the first lumen L1, and the second tube 120 having the second lumen L2. The first tube 110 has the first inner layer 111 corresponding to the innermost layer of the first lumen L1, the first outer layer 112 surrounding the first inner layer 111, and the first reinforcing layer 113 sandwiched between the first inner layer 111 and the first outer layer 112.

In the catheter 10 according to the present embodiment, the first reinforcing layer 113 sandwiched between the first inner layer 111 and the first outer layer 112 can improve the strength of the first tube 110 having the first lumen L1 as described above. This can, for example, prevent a guide wire from unintentionally protruding out of the first lumen L1, or can prevent a guide wire from being stuck due to a collapse of the first lumen.

The catheter 10 according to the present embodiment further comprises the connection layer 130. The connection layer 130 covers at least a portion of the outer peripheral surfaces of the first tube 110 and the second tube 120 to connect the first tube 110 and the second tube 120. The melting point of the connection layer 130 is lower than those of the first inner layer 111 and the first outer layer 112 for first tube 110. Therefore, the catheter 10 according to the present embodiment can prevent the first inner layer 111 and the first outer layer 112 for the first tube 110 from becoming thinner due to welding, effectively increasing the strength of the first tube 110, and can also prevent the first reinforcing layer 113 sandwiched between the first inner layer 111 and the first outer layer 112 from being exposed.

Moreover, in the catheter 10 according to the present embodiment, the outer periphery of the catheter 10 is elliptical in a transverse section. In the catheter 10 according to the present embodiment, this can improve the passability through a lesion site and the operability of a concomitantly-used device as compared to a configuration in which an outer periphery is circular in a transverse section.

Furthermore, in the catheter 10 according to the present embodiment, the first reinforcing layer 113 is a coil. Therefore, in the catheter 10 according to the present embodiment, the coil as the first reinforcing layer 113 can improve the strength of the first tube 110 while ensuring the flexibility required for the catheter 10.

In the catheter 10 according to the present embodiment, the second tube 120 has the second inner layer 121 corresponding to the innermost layer of the second lumen L2, the second outer layer 122 surrounding the second inner layer 121, and the second reinforcing layer 123 sandwiched between the second inner layer 121 and the second outer layer 122. Therefore, in the catheter 10 according to the present embodiment, the second reinforcing layer 123 sandwiched between the second inner layer 121 and the second outer layer 122 can improve the strength of the second tube 120 having the second lumen L2. This can, for example, prevent a guide wire from unintentionally protruding out of the second lumen L2, or can prevent a guide wire from being stuck due to a collapse of the second lumen L2.

### B. Modified Examples:

The technology disclosed herein is not limited to those embodiments as described above, and can be modified to various forms For example, the following modifications are possible.

The configurations of the catheter 10 in the above embodiments are merely illustrative, and can be modified in various ways. For example, in the above embodiments, the first reinforcing layer 113 for the first tube 110 and the second reinforcing layer 123 for the second tube 120 are sparsely wound coils, but the first reinforcing layers 113 and the second reinforcing layer 123 may be tightly wound coils or may be in the forms other than coils (such as braids or tubes).

In the above embodiments, the outer periphery of the catheter 10 in a transverse section is elliptical, but the outer periphery may have a different shape (for example, a circular shape). In the above embodiments, the outer and inner diameters of the first tube 110 and the second tube 120 are substantially the same to each other, but they may be different from each other.

In the above embodiments, both the first tube 110 and the second tube 120 are configured each to have a reinforcing layer sandwiched between an inner layer and an outer layer, but only one of the first tube 110 and the second tube 120 may have such a configuration.

In the above embodiments, the catheter 10 has two lumens, but the catheter 10 may have three or more lumens. In such a case, the strength of the resulting tube can be improved when at least one of the three or more lumens is configured to have a reinforcing layer sandwiched between an inner layer and an outer layer. At least any one of the lumens may not be for a guide wire. For example, a certain lumen may serve as a flow path of a fluid (for example, a drug liquid).

According to the above embodiments, the core wire 50 is embedded in the connection layer 130, but the core wire 50 may be omitted.

In the above embodiments, the technology disclosed herein is described with reference to the catheter 10 to be inserted into a blood vessel as an example, but the technology disclosed herein can equally be applicable to catheters in general to be inserted into a biological lumen (digestive organs such as bile duct, gallbladder, pancreas, esophagus, duodenum, small intestine, and large intestine; blood vessel; urinary tract; trachea; and the like).

### REFERENCE SIGNS LIST

10: Catheter, 20: Shaft, 30: Distal Tip, 40: Connector, 50: Core Wire, 110: First Tube, 111: First Inner Layer, 112: First Outer Layer, 113: First Reinforcing Layer, 116: Opening, 117: Opening, 120: Second Tube, 121: Second Inner Layer, 122: Second Outer Layer, 123: Second Reinforcing Layer, 126: Opening, 127: Opening, 130: Connection Layer, L1: First lumen, L2: Second lumen

## Claims

1. A catheter (10) comprising:
a first tube (110) having a first lumen, and
a second tube (120) having a second lumen, wherein the first tube (110) includes
an inner layer corresponding to an innermost layer of the first lumen,
an outer layer surrounding the inner layer, and
a reinforcing layer sandwiched between the inner layer and the outer layer, **characterized in that**
the catheter (10) further comprises
a connection layer (130) covering at least a portion of the outer peripheral surfaces of the first tube (110) and the second tube (120) to connect the first tube (110) and the second tube (120), and
a core wire (50) embedded in the connection layer (130),
wherein the melting point of the connection layer (130) is lower than those of the inner layer and the outer layer, and
wherein in a cross section of the catheter (10), the first tube (110) and the second tube (120) are exposed to outside of the connection layer (130) at both ends in a direction where the first tube (110) and the second tube (120) are aligned side by side.

2. The catheter (10) according to claim 1, wherein
an outer periphery of the catheter (10) is elliptical in a transverse section.

3. The catheter (10) according to claim 1 or 2, wherein
the reinforcing layer is a coil.

4. The catheter (10) according to any one of claims 1 to 3, wherein
the reinforcing layer is a first reinforcing layer (113),
the inner layer is a first inner layer (111),
the outer layer is a first outer layer (112),
the second tube (120) includes
a second inner layer (121) corresponding to an innermost layer of the second lumen,
a second outer layer (122) surrounding the second inner layer (121), and
a second reinforcing layer (123) sandwiched between the second inner layer (121) and the second outer layer (122).

## Patentansprüche

1. Katheter (10), mit
einem ersten Rohr (110) mit einem ersten Lumen und
einem zweiten Rohr (120) mit einem zweiten Lumen, wobei das erste Rohr (110) aufweist:
eine Innenschicht, die einer innersten Schicht des ersten Lumens entspricht,
eine die Innenschicht umgebende Außenschicht und
eine Verstärkungsschicht, die zwischen der Innenschicht und der Außenschicht angeordnet ist, **dadurch gekennzeichnet, dass**
der Katheter (10) ferner aufweist
eine Verbindungsschicht (130), die zumindest einen Abschnitt der äußeren Außenflächen des ersten Rohrs (110) und des zweiten Rohrs (120) bedeckt, um das erste Rohr (110) und das zweite Rohr (120) zu verbinden, und
einen in die Verbindungsschicht (130) eingebetteten Kerndraht (50),
wobei der Schmelzpunkt der Verbindungsschicht (130) niedriger ist als der der Innenschicht und der Außenschicht, und
wobei in einem Querschnitt des Katheters (10) das erste Rohr (110) und das zweite Rohr (120) an beiden Enden in einer Richtung, in der das erste Rohr (110) und das zweite Rohr (120) nebeneinander ausgerichtet sind, außerhalb der Verbindungsschicht (130) freiliegen.

2. Katheter (10) gemäß Anspruch 1, wobei
ein Außenumfang des Katheters (10) im Querschnitt elliptisch ist.

3. Katheter (10) gemäß Anspruch 1 oder 2, wobei
die Verstärkungsschicht eine Spirale ist.

4. Katheter (10) gemäß einem der Ansprüche 1 bis 3, wobei
die Verstärkungsschicht eine erste Verstärkungsschicht (113) ist,
die Innenschicht eine erste Innenschicht (111) ist,
die Außenschicht eine erste Außenschicht (112) ist,
das zweite Rohr (120) aufweist:
eine zweite Innenschicht (121), die einer innersten Schicht des zweiten Lumens entspricht,
eine zweite Außenschicht (122), die die zweite Innenschicht (121) umgibt, und
eine zweite Verstärkungsschicht (123), die zwischen der zweiten Innenschicht (121) und der zweiten Außenschicht (122) angeordnet ist.

## Revendications

1. Cathéter (10) comprenant :
un premier tube (110) doté d'une première lumière, et
un deuxième tube (120) doté d'une deuxième lumière, dans lequel le premier tube (110) inclut
une couche interne correspondant à une couche la plus interne de la première lumière,
une couche externe entourant la couche interne, et
une couche de renforcement prise en sandwich entre la couche interne et la couche externe, **caractérisée en ce que**
le cathéter (10) comprend en outre
une couche de connexion (130) recouvrant au moins une partie des surfaces périphériques extérieures du premier tube (110) et du deuxième tube (120) pour relier le premier tube (110) et le deuxième tube (120), et
un fil conducteur (50) intégré dans la couche de connexion (130),
dans lequel le point de fusion de la couche de connexion (130) est inférieur à ceux de la couche interne et de la couche externe, et
dans lequel, dans une coupe transversale du cathéter (10), le premier tube (110) et le deuxième tube (120) sont exposés à l'extérieur de la couche de connexion (130) aux deux extrémités dans une direction dans laquelle le premier tube (110) et le deuxième tube (120) sont alignés côte à côte.

2. Cathéter (10) selon la revendication 1, dans lequel
la périphérie extérieure du cathéter (10) est elliptique en coupe transversale.

3. Cathéter (10) selon la revendication 1 ou 2, dans lequel
la couche de renforcement est une bobine.

4. Cathéter (10) selon l'une quelconque des revendications 1 à 3, dans lequel
la couche de renforcement est une première couche de renforcement (113),
la couche interne est une première couche interne (111),
la couche externe est une première couche externe (112),
le deuxième tube (120) inclut
une deuxième couche interne (121) correspondant à une couche la plus interne de la deuxième lumière,
une deuxième couche externe (122) entourant la deuxième couche interne (121), et
une deuxième couche de renforcement (123) prise en sandwich entre la deuxième couche interne (121) et la deuxième couche externe (122).
